(19) Europäisches Patentamt / European Patent Office / Office européen des brevets

(11) **EP 3 617 779 B1**

(12) **EUROPEAN PATENT SPECIFICATION**

(45) Date of publication and mention
of the grant of the patent:
**21.12.2022 Bulletin 2022/51**

(21) Application number: **17907119.6**

(22) Date of filing: **13.07.2017**

(51) International Patent Classification (IPC):
**G02F 1/1335** (2006.01)    **G02B 26/02** (2006.01)
**A61F 9/06** (2006.01)

(52) Cooperative Patent Classification (CPC):
**G02B 26/023; A61F 9/061; A61F 9/065;**
**G02B 27/281; G02F 1/133528;** G02F 1/133531;
G02F 1/13471

(86) International application number:
**PCT/CN2017/092793**

(87) International publication number:
**WO 2018/196174 (01.11.2018 Gazette 2018/44)**

(54) **AUTOMATIC LIGHT CHANGING WELDING GLASS FILTER AND AUTOMATIC LIGHT CHANGING WELDING FACE MASK**

AUTOMATISCHER LICHTWECHSELNDER SCHWEISSGLASFILTER UND AUTOMATISCHE LICHTWECHSELNDE SCHWEISSGESICHTSMASKE

FILTRE DE VERRE DE SOUDAGE À CHANGEMENT DE LUMIÈRE AUTOMATIQUE ET MASQUE FACIAL DE SOUDAGE À CHANGEMENT DE LUMIÈRE AUTOMATIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.04.2017 CN 201710299893**

(43) Date of publication of application:
**04.03.2020 Bulletin 2020/10**

(73) Proprietor: **Wuhan Welhel Photoelectric Co. Ltd**
**Wuhan, Hubei 430023 (CN)**

(72) Inventor: **HUANG, Shangyou**
**Wuhan**
**Hubei 430023 (CN)**

(74) Representative: **Prinz & Partner mbB**
**Patent- und Rechtsanwälte**
**Rundfunkplatz 2**
**80335 München (DE)**

(56) References cited:
**CN-A- 106 361 495**      **CN-U- 202 128 605**
**CN-U- 202 904 170**      **CN-U- 204 618 562**
**US-A- 5 405 184**          **US-A- 5 515 186**
**US-A1- 2004 011 603**  **US-A1- 2014 320 771**

## Description

### FIELD

Embodiments of the present disclosure generally relate to the field of welding protection, and more particularly relate to an auto-darkening filter and an auto-darkening welding helmet.

### BACKGROUND

Welding arc always occurs during a metal welding process. The welding arc is a phenomenon of gas discharge between an electrode and a weldment, which contains a considerable amount of intense visible light, infrared rays, and ultraviolet rays. Optical radiation from the intense visible light, infrared rays and ultraviolet rays causes impairment to the eyes of a welder.

The luminosity of visible light in welding arc may be 10,000 times higher than the luminosity of light bearable to human eyes. Such intense visible light may burn retinas and cause retinitis; in such a case, the welder will feel a pain in eyes and have a blurred vision with a dark point in the center; what's worse, the welder will have a temporary blindness. Repetitive exposure of intense visible light in a long term will gradually degenerate the eyesight.

The infrared rays in the welding arc impair the eyes in a chronic course. With excessive absorption of infrared rays in a long term, the lens would have a reduced elasticity, a difficult adjustability, and a degenerated eyesight; in serious cases, the lens becomes opaque, causing impairment to the eyesight. After the ultraviolet rays in the welding arc are irradiated into human eyes, keratitis and conjunctivitis would be caused; the resulting "electric ophthalmia" is listed a major occupational disease, only second to pneumoconiosis. The ultraviolet rays and infrared rays could cause permanent impairment to human eyes.

A welding helmet is used for protecting a welder from occupational hazards in welding. It may not only protect the eyes of the welder, but also facilitate accurate positioning and efficient welding during a welding process. An earlier welding helmet has a black glass mounted on a portable protective helmet to protect eyes, which requires shading the eyes at the instant of arc striking; if the welder fails to act timely, his/her eyes may be easily burned and the welding quality is affected.

An auto-darkening welding helmet emerges with the advancement of technology. Provided with an auto-darkening filter (ADF), the auto-darkening welding helmet enables an auto-darkening function. The ADF may provide a welder a bright and clear view before an arc is struck, such that the welder may precisely locate a welding spot through the ADF. Once the arc is struck, the ADF may automatically and quickly become dark without manual shading by the welder; in this way, not only the intensity of the visible light is reduced, the ultraviolet rays and infrared rays in the arc may also be effectively blocked, to thereby prevent burning the eyes.

A conventional ADF generally comprises a liquid crystal sheet, an upper polarizing sheet and a lower polarizing sheet which are separately fixed on two sides of the liquid crystal sheet, and a driving circuit driving the liquid crystal sheet, wherein the upper polarizing sheet and the lower polarizing sheet are fixed on two sides of the liquid crystal sheet via a pressure-sensitive adhesive; the driving circuit applies a vertical electrical field, such that the vertical electrical field controls rotation of the liquid crystal molecules in a liquid crystal layer to change a transmittance of an incident ambient light.

Take the most common twisted nematic (TN) liquid crystal sheet as an example, when the driving circuit does not apply a voltage, light having passed through the upper polarizing sheet turns to be a polarized light in a vertical direction; after the polarized light passes through the liquid crystal sheet which is not energized and thus maintains an original arrangement state, thanks to the optical anisotropy of the liquid crystal and the arrangement of the liquid crystal molecules, the polarization direction changes from vertical to horizontal; as the polarized light in the horizontal direction is consistent with a transmission axis direction of the lower polarizing sheet, the light may be transmitted out, and the auto-darkening filter 1 exhibits a bright state.

When the driving circuit applies a certain voltage to the liquid crystal sheet, the TN liquid crystal molecules change from a twisted arrangement state to a vertical arrangement state; at this point, the polarization direction of the linear polarizing light incident through the upper polarizing sheet does not change, causing the polarized light direction to be vertical to the transmission axis direction of the lower polarizing sheet; thus, the light is occluded and cannot be transmitted out of the lower polarizing sheet; consequently, the auto-darkening filter 1 exhibits a dark state.

The ADF is in a bright state before arc striking, such that the welder may prepare for welding through the ADF; upon occurrence of a welding arc, the ADF is automatically darkened into a dark state, thereby preventing the welder's eyes from being impaired by optical radiation.

Conventional ADFs generally use a single liquid crystal sheet or a plurality of stacked liquid crystal sheets, wherein a driving voltage is applied to the liquid crystal to adjust the transmittance of an ambient light incident into the ADF.

When the ADF is provided with a single liquid crystal sheet, the driving circuit works in a high-voltage low-frequency (20-40V, 0.01-1 Hz) state. Although the ADF with a single liquid crystal sheet has a low cost and provides a good view under a relatively high transmittance, the fixed driving voltage results in that the transmittance of the ADF is fixed in the dark state, such that the transmittance of the dark-state ADF cannot be correspondingly adjusted based on a welding environment; consequently, a potential consequence of which is that the transmittance of the ADF is over high and what is transmitted through the ADF is still the intense visible light, which may still hurt the welder's eyes; or the transmittance of the ADF is too low, which leads to a too dark view, such that the welder cannot spot the welding point clearly, thereby affecting the efficiency of the welding work.

Currently, there also exists a practice of leveraging a circuit to adjust the transmittance of the single liquid crystal sheet; however, such a practice provides a poor viewing angle property and a narrow transmittance adjustability (the minimum transmittance is too large), such that it basically cannot satisfy the welding protection standards in various countries.

When the ADF is provided with two or more parallel arranged liquid crystal sheets, the driving circuit works in a low-voltage high-frequency (2-15V, 40~100Hz) state. Although the ADF with multiple liquid crystal sheets realizes adjustment of the transmittance of light in the dark state by partially changing the twisted state of the liquid crystal molecules, such ADFs have a high cost, a large size, a large weight, and a complex control, which does not facilitate manufacturing.

From US 5 515 186 A an optical system for a welding helmet is known, the system including two identical optical components, each having a polarizer and a further polarizer (analyzer). To improve the transmission characteristics of the system, the transmission axes of the optical components are arranged at angle to each other.

Moreover, US 5 405 184 A shows a cover of a vehicle roof, wherein the light light-transmissibility of the cover is variable.

US 2004/011 603 A1 further discloses a vehicle brake control apparatus with a motor and a movement conversion portion that reduces a speed of a revolution of the motor from a low gear ratio to a high gear ratio in at least two steps.

Therefore, there is an urgent need to develop a novel auto-darkening filter to solve the problems above.

## SUMMARY

An object of the present disclosure is to provide an auto-darkening filter to solve the problems existing in conventional auto-darkening filters, such as a poor viewing angle property, an over high power consumption, a high cost, and a poor transmittance adjustment effect or even adjustment incapability for a single liquid crystal sheet.

To achieve the object above, the present disclosure provides an auto-darkening filter according to claim 1.

Preferably, an initial position of the rotatable movable polarizing sheet is a position where transmission axis directions of the two polarizing sheets are perpendicular or parallel to each other, and with the initial position as a reference, an angle of rotation of the rotatable movable polarizing sheet is -5°~5°

Preferably, an initial position of the rotatable movable polarizing sheet is a position where transmission axis directions of the two polarizing sheets are perpendicular or parallel to each other, and with the initial position as a reference, an angle of rotation of the movable polarizing sheet is 0°-5°.

Preferably, the auto-darkening filter further comprises a second crystal sheet, wherein the first and second liquid crystal sheets are arranged in parallel, and a third polarizing sheet. The first liquid crystal sheet is sandwiched between the first polarizing sheet and the second polarizing sheet, and the second liquid crystal sheet is sandwiched between the second polarizing sheet and the third polarizing sheet. At least one of the first polarizing sheet and the second polarizing sheet and the third polarizing sheet is the rotatable movable polarizing sheet.

Preferably, the angle adjusting device comprises an angle adjusting knob, a disc-shaped cam connected to the angle adjusting knob, and an elastic element; wherein the disc-shaped cam abuts against one side of the rotatable movable polarizing sheet, and the elastic element is connected to the other side of the movable polarizing sheet.

Preferably, the elastic element is a spring.

Preferably, a visible light transmittance of the auto-darkening filter is in a range of $10^{-6}$ to $10^{-1}$.

Preferably, a driving voltage of the driving circuit is in a range of 20V to 40V.

Preferably, a driving frequency of the driving circuit is in a range of 0.01Hz to 1Hz.

The present disclosure further provides an auto-darkening welding helmet, comprising the auto-darkening filter mentioned above.

Compared with the prior art, the auto-darkening filter and the auto-darkening welding helmet according to the present disclosure provide the following advantages:

1. In the present disclosure, at least one polarizing sheet is configured as a rotatable movable polarizing sheet, which enables adjustment of the angle between the transmission axes of two polarizing sheets. An angle of rotation is selected based on welding environments such as a welding approach and a welding current in use. By rotating the movable polarizing sheet 0°~5°, the visible light transmittance of the auto-darkening filter may vary within a range of $10^{-6}$ to $10^{-1}$ such that the liquid crystal sheet may be flexibly adjusted to an appropriate shade number. Therefore, the present disclosure enables a large transmittance adjustable range only with a small adjustment angle. Moreover, with a single liquid crystal sheet, the present disclosure may achieve a viewing angle effect that is conventionally achieved by two liquid crystal sheets; meanwhile, the driving circuit works in a high-voltage and low-frequency state, which may lower the cost, reduce power consumption of the circuit, and meanwhile improve the viewing angle response property of liquid crystal;

2. When the auto-darkening filter provided by the present disclosure adopts two or more liquid crystal sheets, at least one polarizing sheet is configured as an angle rotation-enabled movable polarizing sheet. In this way, a smaller angle rotation enables adjustment to a different shade number. Besides, the auto-darkening filter provides a better viewing angle. Moreover, the high-voltage low-frequency working state of the driving circuit further reduces the cost.

## BRIEF DESCRIPTION OF THE DRAWINGS

Various other advantages and benefits will become clearer to those skilled in the art through detailed description of the preferred embodiments below. The accompanying drawings are only used for the purpose of preferred embodiments and should not be construed as a limitation to the present disclosure. In the drawings:

Fig. 1 shows a structural schematic diagram of an auto-darkening filter according to a first preferred embodiment of the present disclosure;

Fig. 2 shows a first cross-sectional schematic diagram of the auto-darkening filter in Fig. 1;

Fig. 3 shows a second cross-sectional schematic diagram of the auto-darkening filter in Fig. 1;

Fig. 4 shows a cross-sectional schematic diagram of an auto-darkening filter according to a second preferred embodiment of the present disclosure;

Fig. 5 shows a structural schematic diagram of an auto-darkening welding helmet according to a preferred embodiment of the present disclosure.

Reference Numerals:

| 1- | auto-darkening filter | 11- | liquid crystal sheet, |
|---|---|---|---|
| 111 - | upper-layer glass substrate | 113- | lower-layer glass substrate |
| 115- | liquid crystal layer | 13- | polarizing sheet, |
| 131- | first polarizing sheet | 133- | second polarizing sheet, |
| 135- | third polarizing sheet | 15 - | driving circuit |
| 17 - | angle adjusting device | 171 - | angle adjusting knob |
| 173 - | disc-shaped cam | 175- | elastic element. |

## DETAILED DESCRIPTION OF EMBODIMENTS

The present disclosure provides a plurality of applicable innovative concepts which may be extensively embodied in the context. The specific examples described in the embodiments of the present disclosure only serve as exemplary illustrations of the preferred embodiments of the present disclosure, not constituting a limitation to the scope of the present disclosure.

Hereinafter, the present disclosure will be illustrated in further detail through preferred embodiments with reference to the accompanying drawings.

First, the terms in the present disclosure will be explained:

In electromagnetic spectrum, the wavelengths ranging from 380nm to 780nm are defined as the visible light range, i.e., an optical radiation range that may be detected by human eyes; the wavelength range under 380nm is defined as an ultraviolet waveband, while the wavelength range above 780nm is defined as the infrared waveband.

In the welding industry, in accordance with the national industrial standard GB/T3609.1-2008 Occupational Eye and Face Protection-Welding Protection Part 1: Welding Protector, a welder's face shield refers to a face shield equipped with an appropriate filter, intended for protecting eyes and face during a welding operation.

A dark number of a filter is defined as a shade number N, wherein a relationship expression between the shade number N and the visible light transmission ratio $\tau_v$ is shown in Equation (1):

$$N = 1 + \frac{7}{3} \lg\left(\frac{1}{\tau_v}\right)$$

(Equation 1)

Different visible light transmission ratios (visible light transmittance) corresponding to different shade numbers may be calculated from equation (1), as shown in Table 1.

Table 1 Visible Light Transmission Ratios Corresponding to Different Shade Numbers

| Shade Number | Visible Light Transmission Ratios | |
|---|---|---|
| | 380nm~780nm | |
| | Maximum | Minimum |
| 3 | 0.178 | 0.085 |
| 4 | 0.085 | 0.032 |
| 5 | 0.032 | 0.012 |
| 6 | 0.012 | 0.0044 |
| 7 | 0.0044 | 0.0016 |
| 8 | 0.0016 | 0.00061 |
| 9 | 0.00061 | 0.00023 |
| 10 | 0.00023 | 0.000085 |
| 11 | 0.000085 | 0.000032 |
| 12 | 0.000032 | 0.000012 |
| 13 | 0.000012 | 0.0000044 |

It may be seen from Table 1 that for different shade numbers, the visible light transmission ratios differ greatly. For example, when the shade number is 3, the visible light transmission ratio is relatively high, reaching $10^{-1}$, namely, attenuating the light to 1/10, which indicates that more visible light is transmitted through the filter; at this point, the view of the welder is relatively bright. When the shade number is 13, the visible light transmission ratio is very low, reaching $10^{-6}$, i.e., attenuating the light to one millionth, which indicates that very little visible light is transmitted through the light filter; at this point, intense visible light may be blocked from hurting the welder's eyes. In the welding industry, dependent on different welding environments, the shade number of the optical filter upon arc striking is appropriately 9~13.

**Example 1**

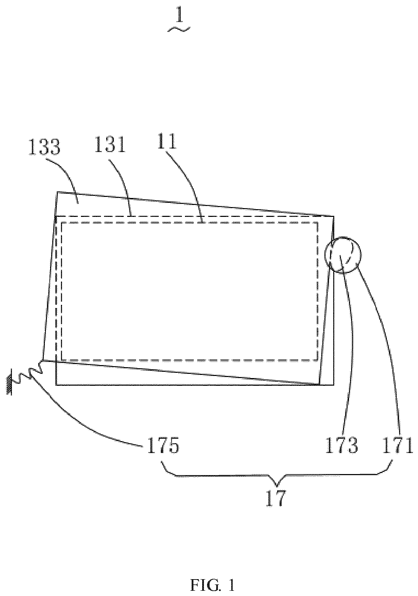

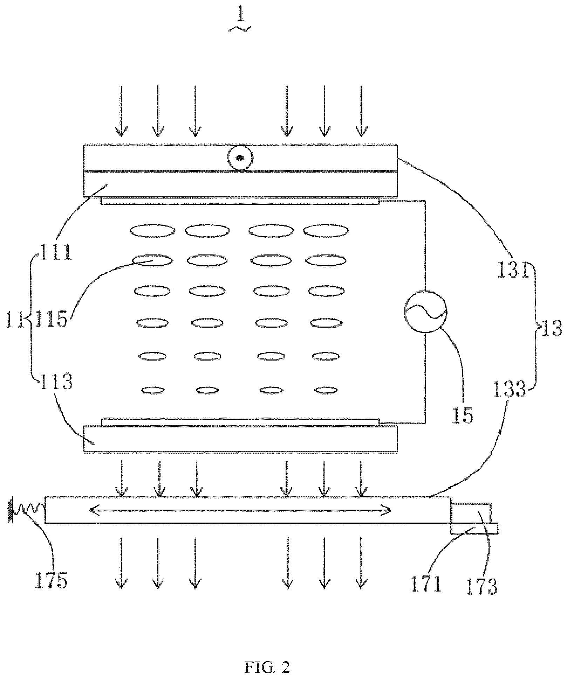

As shown in Figs. 1 and 2, this example provides an auto-darkening filter 1, comprising: a liquid crystal sheet 11, two polarizing sheets 13 separately disposed on two sides of the liquid crystal sheet 11, a driving circuit 15 applying a voltage to drive the liquid crystal sheet 11, and an angle adjusting device 17, wherein one polarizing sheets 13 is configured as a rotatable movable polarizing sheet, for changing an angle between transmission axes of the two polarizing sheets 13, the angle being adjusted by the angle adjusting device 17.

The liquid crystal sheet 11 comprises an upper layer glass substrate 111, a lower layer glass substrate 113, and a liquid crystal layer 115 sandwiched between the upper layer glass substrate 111 and the lower layer glass substrate 113. In this embodiment, one liquid crystal sheet 11 is provided, which has a rectangular shape. Of course, the number of the liquid crystal sheets 11 may be two or more, and the shape is not limited to rectangle, which may optionally be a round or other shape.

The liquid crystal sheet has a variety of types. The liquid crystal sheet 11 provided by the present disclosure takes the most common twisted nematic (TN) liquid crystal sheet as an example. In other embodiments, the liquid crystal sheet 13 may also be other types of liquid crystal sheet, e.g., a vertical alignment (VA) liquid crystal sheet, an in-plane switching (IPS) liquid crystal sheet, and a fringe field switching (FFS) liquid crystal sheet, etc., as long as it may control rotation of liquid crystal molecules via electrical signals.

The polarizing sheet 13 comprises a first polarizing sheet 131 and a second polarizing sheet 133, which are arranged in parallel at intervals, and the second polarizing sheet 133 is configured as a rotatable movable polarizing sheet. The shape of the polarizing sheet 13 is preferably rectangular, but is not limited thereto, as long as it is fitted with the shape of the liquid crystal sheet 11 such that when the second polarizing sheet 133 is rotating, the polarizing sheet 13 may completely occlude the liquid crystal sheet 11.

The first polarizing sheet 131 is disposed on a surface of the upper layer glass substrate 111 distant from the liquid crystal layer 115, and the first polarizing sheet 131 and the upper layer glass substrate 111 are securely connected via a pressure-sensitive adhesive. Of course, they may also be connected in other manners, as long as the first polarizing sheet 131 can be securely connected with the upper layer glass substrate 111.

The second polarizing sheet 133 is fitted to a surface of the lower layer glass substrate 113 distant from the liquid crystal layer 115, the "fit" here meaning that a surface of the second polarizing sheet 133 abuts the surface of the lower layer glass substrate 113, such that the second polarizing sheet 133 may rotate relative to the first polarizing sheet 133 on a plane where the second polarizing sheet 133 is located.

The second polarizing sheet 133 is configured as a rotatable movable polarizing sheet, for changing an angle between transmission axes of the first polarizing sheet 131 and the second polarizing sheet 133; the light transmittance varies with the angle so as to further change the shade number of the auto-darkening filter 1 in a dark state.

An initial position of the second polarizing sheet 133 is a position where the transmission axis of the first polarizing sheet 131 is vertical to the transmission axis of the second polarizing sheet 133. Of course, the initial position of the second polarizing sheet 133 may optionally be a position where the transmission axis of the first polarizing sheet 131 is parallel to the transmission axis of the second polarizing sheet 133, or is not vertical thereto, but has a specific angle therewith. Namely, the angle between the transmission axis direction of the first polarizing sheet 131 and the transmission axis direction of the second polarizing sheet 133 is preset before the second polarizing sheet 133 rotates an angle, and then the shade number is further adjusted by rotating the second polarizing sheet 133 an angle.

The second polarizing sheet 133 rotates different angles clockwise or counterclockwise with the initial position as a reference; the visible light transmittances of the auto-darkening filter 1 in a range from 380nm to 780nm are measured using a spectrometer which measures an optical density as high as OD7; and then, shade numbers corresponding to different visible light transmittances may be calculated from the equation (1), results of which are shown in Table 2 below.

Table 2 Shade Numbers Corresponding to Different Angles of Rotational

| Rotational Angle | Visible Light Transmittance | Shade Number |
|---|---|---|
| -5° | 0.1330% | 7.708 |
| -4° | 0.0450% | 8.792 |
| -3° | 0.0240% | 9.787 |
| -2° | 0.0070% | 10.648 |
| -1° | 0.0032% | 11.488 |
| -0.5° | 0.0011% | 12.571 |
| 0° | 0.0005% | 13.402 |
| 0.5° | 0.0016% | 12.190 |
| 1° | 0.0037% | 11.341 |
| 2° | 0.0055% | 10.939 |
| 3° | 0.0200% | 9.595 |
| 4° | 0.0600% | 8.516 |
| 5° | 0.1250% | 7.771 |

It may be seen from Table 2 that with the initial position as a reference, when the second polarizing sheet 133 rotates an angle in a range of -5° to 5°, the visible light transmittance of the auto-darkening filter 1 ranges from $10^{-6}$ to $10^{-3}$. In other words, the intense visible light may be attenuated to a range of one thousandth to one millionth, i.e., a shade number in a range of 7 to 13. This indicates that the auto-darkening filter 1 provided by the present disclosure has a wider adjustment range and is applicable to most welding environments, which enables selection of appropriate shade numbers for different welding environments, thereby preventing the eyes from being impaired by a too high or too low transmittance.

Moreover, irrespective of clockwise rotation of 0°~5° or counterclockwise rotation of 0°~-5°, the second polarizing sheet 133 may perform a dark-state shade number adjustment in a range of 7 to 13, which indicates that the objective of shade number adjustment may be attained by unidirectional rotation of the second polarizing sheet 133; therefore, when the second polarizing sheet 133 serves as the movable polarizing sheet, the rotational angle is preferably 0°~5°; further, when the second polarizing sheet 133 rotates an angle in a range of 0° to 5°, particularly of 0°~1°, each rotation of 0.5° will decrease the shade number by 1, which indicates that a very small rotational angle may lead to a very large amplitude of shade number change, which thereby provides a better adjustment effect; therefore, the angle of rotation of the second polarizing sheet 133 is preferably in a range of 0° to 3°.

In this embodiment, the first polarizing sheet 131 is a fixed polarizing sheet, while the second polarizing sheet 133 is a rotatable movable polarizing sheet. Of course, the first polarizing sheet 131 may serve both as a fixed polarizing sheet and a rotatable movable polarizing sheet. Or the first polarizing sheet 131 serves as a rotatable movable polarizing sheet while the second polarizing sheet 133 serves as a fixed polarizing sheet. The present disclosure has no limitation thereto, as long as the angle between the transmission axis of the first polarizing sheet 131 and the transmission axis of the second polarizing sheet 133 may be changed.

The driving circuit 15 has a driving voltage of 20V to 40V (peak value) and a driving frequency of 0.01Hz~1Hz; therefore, its working state is high-voltage low-frequency. Compared with the existing low-frequency high-voltage working state, the high-voltage low-frequency work state may reduce power consumption of the circuit, has a lower cost, and meanwhile may improve the viewing angle response property of the liquid crystal.

Further, as shown in Fig. 1, the angle adjusting device 17 is connected to the polarizing sheet 13, for adjusting the angle between the transmission axis of the first polarizing sheet 131 and the transmission axis of the second polarizing sheet 133.

Exemplary, the angle adjusting device 17 comprises an angle adjusting knob 171, a disc-shaped cam 173 connected to the angle adjusting knob 171, and an elastic element 175; wherein the disc-shaped cam 173 abuts against one side of the second polarizing sheet 133, and the elastic element 175 is connected to the other side of the second polarizing sheet 133. The elastic element 175 is preferably a spring.

The disc-shaped cam 173 is a cam which has a radial profile dimension variation and rotates about its axis, thereby providing a simple and compact structure and a convenient design. Specifically, a side face of the second polarizing sheet 133 abuts against one side of the disc-shaped cam 173 with a smaller cam profile radius, wherein the "abut against" means that the side face of the second polarizing sheet 133 contacts with and abuts the side face of the disc-shaped cam 173 with a smaller cam profile radius.

Upon arc striking, the auto-darkening filter 1 automatically becomes dark to protect eyes; the user controls the angle adjusting device 17 to adjust the angle between the transmission axis of the first polarizing sheet 131 and the transmission axis of the second polarizing sheet 133 so as to reach an appropriate position. In this way, a clear enough view may be provided, such that the welder may spot the welding point clearly; besides, the intensity of the visible light may be controlled from hurting the welder's eyes.

The initial position of the second polarizing sheet 133 is that the transmission axis of the first polarizing sheet 131 is perpendicular to the transmission axis of the second polarizing sheet 133. With the angle adjusting device 17, the user may control the angle adjusting knob 171 to rotate clockwise, such that the angle adjusting knob 171 may drive the disc-shaped cam 173 to rotate, and then the disc-shaped cam 173 pushes forwardly the second polarizing sheet 133 to rotate to an appropriate position with the initial position as a reference, wherein the angle of rotation is preferably 0°~5°; meanwhile, the elastic element 175 is stretched. As a frictional force of the angle adjustment knob 171 is greater than an elastic force of the elastic element 175, the angle of rotation is stable and the user may perform a welding operation;

Dependent on different welding environments, when the user needs to resume the position of the second polarizing sheet 133, the user controls the angle adjusting knob 171 to rotate counterclockwise; then, the angle adjusting knob 171 drives the disc-shaped cam 173 to rotate reversely, and then the elastic element 175, thanks to its elastic force, bounces back to the second polarizing sheet 133.

Of course, the angle adjusting device 17 may alternatively have other shapes or structures. There present disclosure has no limitation to the shape of the angle adjusting device 17, as long as it may rotate the movable polarizing sheet to a corresponding angle. The auto-darkening principle of the auto-darkening filter 1 of the TN liquid crystal sheet adopted in this example is described as follows:

As shown in Fig. 2, before arc striking, the user does not perform a welding operation, the driving circuit 15 applies no voltage, the liquid crystal molecules in the liquid crystal layer 115 are parallel to the upper layer glass substrate 111 and the lower layer glass substrate 113, the long axis direction of the liquid crystal molecules, subjected to the action of an alignment film anchoring energy, is parallel to the frictional force direction of the alignment films on the upper layer glass substrate 111 and the lower layer glass substrate 113, while the liquid crystal molecules in respective intermediate layers, subjected to the action of chiral molecules, are sequentially and spirally arranged from the upper layer liquid crystal molecule direction to the lower layer liquid crystal molecule direction. The difference between the frictional force directions of the alignment films of the TN liquid crystal units is generally 90°, such that the angle of the spiral arrangement of the liquid crystal molecules is also 90°. As liquid crystal molecules have a birefringent property, the spirally arranged liquid crystal molecules have an optical rotation effect to light; therefore, after penetrating through the liquid crystal layer, the light polarized state rotates 90°. In this example, the transmission axis direction of the first polarizing sheet 131 is perpendicular to the transmission axis direction of the second polarizing sheet 133, i.e., assuming a 90°; therefore, the transmission axis direction of the first polarizing sheet 131 is parallel to an optical axis direction of the upper layer liquid crystal molecules, and the transmission axis direction of the second polarizing sheet 133 is parallel to the optical axis direction of the lower layer liquid crystal molecules; after the light transmitted through the first polarizing sheet 131 is rotated via the liquid crystal layer 115, the polarizing state rotates 90°, exactly parallel to the transmission axis of the second polarizing sheet 133, such that the light may be transmitted out, and the auto-darkening filter 1 exhibits a bright state.

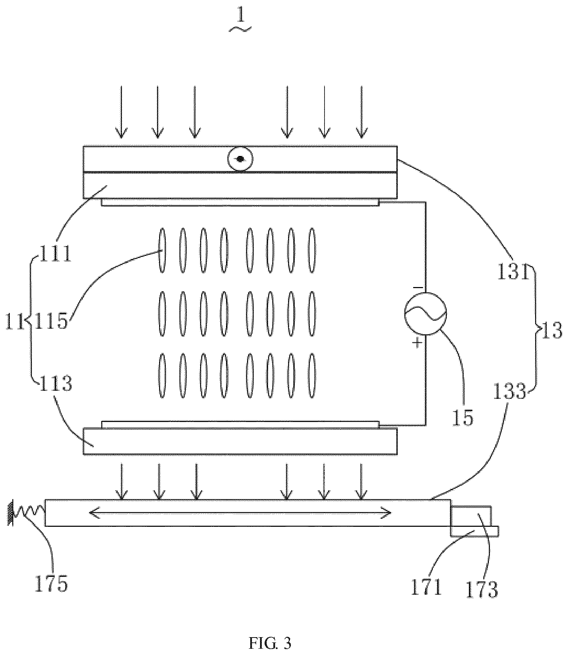

As shown in Fig. 3, after an arc is struck, the arc may be detected by a sensor externally connected to the auto-darkening filter 1; the driving circuit 15 applies a certain voltage between the upper layer glass substrate 111 and the lower layer glass substrate 113; as the TN liquid crystal sheet uses a positive liquid crystal, such that under the action of an electrical field, the long axis direction of the liquid crystal molecules tends to be arranged parallel to the electrical field direction while perpendicular to the upper layer glass substrate 111 and the lower layer glass substrate 113. At this point, because the polarized state optical waves passing through the first polarizing sheet 131 are propagated along the long axis (optical axis) of the liquid crystal molecules, no birefringence occurs, and the polarization direction of the liquid crystal molecules does not change; because the transmission axis of the first polarizing sheet 131 is perpendicular to the transmission axis of the second polarizing sheet 133, light cannot be transmitted out of the second polarizing sheet 133, and the auto-darkening filter 1 exhibits a dark state.

Such a mode, in which the auto-darkening filter 1 is in a bright state when 0 voltage is applied, is referred to as a normal white mode; likewise, when the transmission axis direction of the first polarizing sheet 131 is parallel to the transmission axis direction of the second polarizing sheet 133, if no electrical field is applied, the light having passing through the liquid crystal sheet 11 cannot be transmitted out of the second polarizing sheet 13, such that the auto-darkening filter 1 exhibits a dark state; when the electrical field is applied, the light is transmitted out of the second polarizing sheet 13, and the auto-darkening filter 1 exhibits a bright state, wherein the dark state mode of the auto-darkening filter 1 when the electrical field is 0 is referred to as a normal black mode. Both the normal white mode and the normal black mode are applicable to the present disclosure; however, the normal white mode facilitates power consumption reduction.

Compared with a conventional filter, in which the polarizing sheet is fixed to the outer side of the liquid crystal sheet via a pressure-sensitive adhesive such that it is hard to adjust the light transmittance when the driving circuit applies a fixed voltage, the present disclosure configures the second polarizing sheet 133 as a rotatable movable polarizing sheet, so as to enable adjustment of the angle between the transmission axis of the first polarizing sheet 131 and the transmission axis of the second polarizing sheet 133. An angle of rotation is selected based on welding environments such as a welding approach and a welding current in use. By rotating 0°~5° with the initial position as a reference, the visible light transmittance of the auto-darkening filter may be varied such that the liquid crystal sheet may be flexibly adjusted to an appropriate shade number. Moreover, with a single liquid crystal sheet, the present disclosure may achieve a viewing angle effect that is conventionally achieved by two liquid crystal sheets; meanwhile, the driving circuit works in a high-voltage and low-frequency state, which may lower the cost, reduce power consumption of the circuit, and meanwhile improve the viewing angle response property of liquid crystal.

**Example 2**

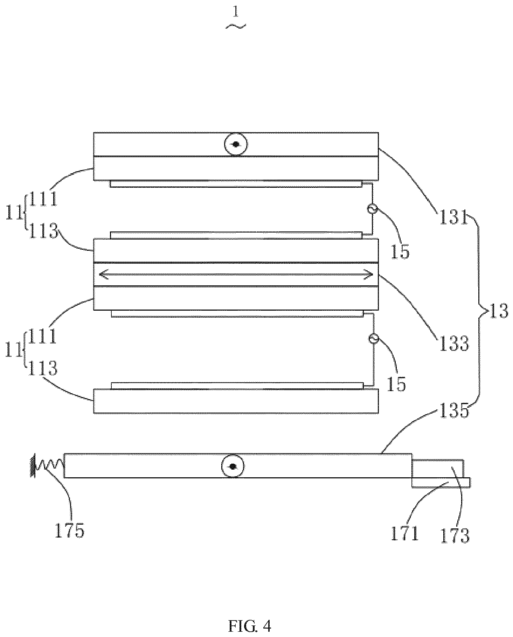

As shown in Fig. 4, the auto-darkening filter provided in this example differs from that provided in example 1 only in providing two liquid crystal sheets 11 and three polarizing sheets 13.

The two liquid crystal sheets 11 are arranged in parallel, a common polarizing sheet 13 being sandwiched therebetween; one polarizing sheet 13 is provided on an outer side of each of the two liquid crystal sheets 11; and at least one of the three polarizing sheets 13 is a rotatable movable polarizing sheet.

Specifically, the polarizing sheet 13 comprises a first polarizing sheet 131, a second polarizing sheet 133, and a third polarizing sheet 135, which are arranged in parallel at intervals; one of the liquid crystal sheets 11 being sandwiched between the first polarizing sheet 131 and the second polarizing sheet 132, and the other liquid crystal sheet 11 being sandwiched between the second polarizing sheet 133 and the third polarizing sheet 135, wherein at least one of the first polarizing sheet 131, the second polarizing sheet 133, and the third polarizing sheet 135 is a rotatable movable polarizing sheet. Due to presence of two liquid crystal sheets 11 and three polarizing sheets 13, the more the rotatable movable polarizing sheets are provided, the more flexible the adjustment is, and the smaller the angle that needs to be adjusted is. The working state of the driving circuit 15 is likewise high-voltage low-frequency.

Further, the positions of the first polarizing sheet 131 and the second polarizing sheet 133 are arranged such that the angle between their transmission axes is a fixed angle, preferably 7°; the third polarizing sheet 135 is configured as a rotatable movable polarizing sheet, via which the angle between the transmission axis of the second polarizing sheet 133 and the transmission axis of the third polarizing sheet 135 is adjusted, preferably in a range of 3° to 7°; then, the linearity degree of transmittance adjustment will increase, and the viewing angle will also be significantly improved.

Compared with a conventional auto-darkening filter which has two liquid crystal sheets and has a low-voltage high-frequency driving manner, the auto-darkening filter 1 provided by the present disclosure adopts a solution of configuring at least one of the polarizing sheets 13 as a rotatable movable polarizing sheet in the case of adopting two liquid crystal sheets 11, which not only enables flexible adjustment of the shade number, but provides a better view with only a smaller angle adjustment; meanwhile, the driving circuit works in a high-voltage low-frequency state, which may effectively reduce costs.

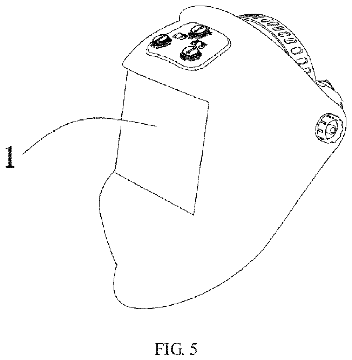

As shown in Fig. 5, the present disclosure further provides an auto-darkening welding helmet. The auto-darkening welding helmet comprises the auto-darkening filter 1, the auto-darkening filter 1 is fixed to the auto-darkening welding helmet via a frame, wherein the auto-darkening welding helmet enables adjustment of a dark state shade number using the auto-darkening filter 1 so as to protect a welder's eyes.

It should be noted that, the examples above are intended to illustrate the present disclosure, not to limit the present disclosure. Without departing from the scope of the claims, those skilled in the art may devise alternative examples. In the claims, no reference numerals placed in the parentheses should be construed as any limitation to the claims. The word "comprise" does not exclude the elements or steps not recited in the claims.

**Claims**

1.  An auto-darkening filter (1) comprising:

    a first liquid crystal sheet (11); two polarizing sheets (13) comprising a first polarizing sheet (131) and a second polarizing sheet (133) and separately disposed on two sides of the first liquid crystal sheet (11);
    a driving circuit (15) driving the first liquid crystal sheet (11); **characterized by** an angle adjusting device (17); wherein
    at least one of the two polarizing sheets (13) is configured as a rotatable movable polarizing sheet (133), for changing an angle between transmission axes of the two polarizing sheets (13), the angle being adjusted by the angle adjusting device (17);
    wherein the shape of the rotatable movable polarizing sheet (13) is fitted with the shape of the first liquid crystal sheet (11) such that when the rotatable movable polarizing sheet (13) is the second polarizing sheet (133) and when the second polarizing sheet (133) is rotating, the rotatable movable polarizing sheet (13) completely occludes the first liquid crystal sheet (11).

2.  The auto-darkening filter according to claim 1, **characterized in that**, an initial position of the rotatable movable polarizing sheet a position where transmission axis directions of the two polarizing sheets (13) are perpendicular or parallel to each other, and with the initial position as a reference, an angle of rotation of the rotatable movable polarizing sheet is -5°~5°

3.  The auto-darkening filter according to claim 2, **characterized in that**, an initial position of the rotatable movable polarizing sheet being a position where transmission axis directions of the two polarizing sheets (13) are perpendicular or parallel to each other, and with the initial position as a reference, the angle of rotation of the rotatable movable polarizing sheet is 0°~5°

4.  The auto-darkening filter according to claim 1 further comprising:

    second liquid crystal sheet (11), wherein the first and second liquid crystal sheets (11) are arranged in parallel, and a third polarizing sheet (13), wherein the first liquid crystal sheet (11) is sandwiched between the first polarizing sheet (131) and the second polarizing sheet (132), and the second liquid crystal sheet (11) is sandwiched between the second polarizing sheet (133) and the third polarizing sheet (135),
    wherein at least one of the first polarizing sheet (131) and the second polarizing sheet (132) and the third polarizing sheet (135) is the rotatable movable polarizing sheet.

5.  The auto-darkening filter according to claim 2, **characterized in that**, the angle adjusting device (17) comprises:

    an angle adjusting knob (171);

a disc-shaped cam (173) connected to the angle adjusting knob (171); and
an elastic element (175); wherein
the disc-shaped cam (173) abuts against one side of the rotatable movable polarizing sheet, and the elastic element (175) is connected to the other side of the rotatable movable polarizing sheet.

6. The auto-darkening filter according to claim 5, **characterized in that**, the elastic element (175) is a spring.

7. The auto-darkening filter according to claim 1, wherein a visible light transmittance of the auto-darkening filter (1) is in a range of $10^{-6}$ to $10^{-1}$.

8. The auto-darkening filter according to claim 1, wherein a driving voltage of the driving circuit (15) is in a range of 20V to 40V.

9. The auto-darkening filter according to claim 1, wherein a driving frequency of the driving circuit (15) is in a range of 0.01Hz to 1Hz.

10. An auto-darkening welding helmet comprising the auto-darkening filter according to any one of claims 1-9.


**Patentansprüche**

1. Selbstverdunkelnder Filter (1) mit:

   einer ersten Flüssigkristallfolie (11),
   zwei Polarisationsfolien (13), die eine erste Polarisationsfolie (131) und eine zweite Polarisationsfolie (133) umfassen und getrennt auf zwei Seiten der ersten Flüssigkristallfolie (11) angeordnet sind,
   einer Ansteuerschaltung (15), die die erste Flüssigkristallfolie (11) ansteuert, **gekennzeichnet durch**
   eine Winkeleinstellvorrichtung (17), wobei
   wenigstens eine der beiden Polarisationsfolien (13) als drehbare bewegliche Polarisationsfolie (133) ausgebildet ist, um einen Winkel zwischen Transmissionsachsen der beiden Polarisationsfolien (13) zu ändern, wobei der Winkel durch die Winkeleinstellvorrichtung (17) eingestellt wird,
   wobei die Form der drehbaren beweglichen Polarisationsfolie (13) so mit der Form der ersten Flüssigkristallfolie (11) zusammenpasst, dass dann, wenn die drehbare bewegliche Polarisationsfolie (13) die zweite Polarisationsfolie (133) ist und wenn sich die zweite Polarisationsfolie (133) dreht, die drehbare bewegliche Polarisationsfolie (13) die erste Flüssigkristallfolie (11) vollständig verdeckt.

2. Selbstverdunkelnder Filter nach Anspruch 1, **dadurch gekennzeichnet, dass** bei einer Ausgangsposition der drehbaren beweglichen Polarisationsfolie, die eine Position ist, bei der die Transmissionsachsenrichtungen der beiden Polarisationsfolien (13) senkrecht oder parallel zueinander sind, und mit der Ausgangsposition als Referenz ein Drehwinkel der drehbaren beweglichen Polarisationsfolie -5°~5° beträgt.

3. Selbstverdunkelnder Filter nach Anspruch 2, **dadurch gekennzeichnet, dass** bei einer Ausgangsposition der drehbaren beweglichen Polarisationsfolie, die eine Position ist, bei der die Transmissionsachsenrichtungen der beiden Polarisationsfolien (13) senkrecht oder parallel zueinander sind, und mit der Ausgangsposition als Referenz der Drehwinkel der drehbaren beweglichen Polarisationsfolie 0°~5° beträgt.

4. Selbstverdunkelnder Filter nach Anspruch 1, ferner mit:

   einer zweiten Flüssigkristallfolie (11), wobei die erste und die zweite Flüssigkristallfolie (11) parallel angeordnet sind, und
   einer dritten Polarisationsfolie (13),
   wobei die erste Flüssigkristallfolie (11) sandwichartig zwischen der ersten Polarisationsfolie (131) und der zweiten Polarisationsfolie (132) angeordnet ist und die zweite Flüssigkristallfolie (11) sandwichartig zwischen der zweiten Polarisationsfolie (133) und der dritten Polarisationsfolie (135) angeordnet ist,
   wobei die erste Polarisationsfolie (131) und/oder die zweite Polarisationsfolie (132) und/oder die dritte Polarisationsfolie (135) die drehbare bewegliche Polarisationsfolie ist.

5. Selbstverdunkelnder Filter nach Anspruch 2, **dadurch gekennzeichnet, dass** die Winkeleinstellvorrichtung (17) Folgendes umfasst:

einen Winkeleinstellknopf (171),
einen scheibenförmigen Nocken (173), der mit dem Winkeleinstellknopf (171) verbunden ist, und
ein elastisches Element (175), wobei
der scheibenförmige Nocken (173) an einer Seite der drehbaren beweglichen Polarisationsfolie anliegt und das elastische Element (175) mit der anderen Seite der drehbaren beweglichen Polarisationsfolie verbunden ist.

**6.** Selbstverdunkelnder Filter nach Anspruch 5, **dadurch gekennzeichnet, dass** das elastische Element (175) eine Feder ist.

**7.** Selbstverdunkelnder Filter nach Anspruch 1, bei dem eine Durchlässigkeit des selbstverdunkelnden Filters (1) für sichtbares Licht in einem Bereich von $10^{-6}$ bis $10^{-1}$ liegt.

**8.** Selbstverdunkelnder Filter nach Anspruch 1, bei dem eine Ansteuerspannung der Ansteuerschaltung (15) in einem Bereich von 20 V bis 40 V liegt.

**9.** Selbstverdunkelnder Filter nach Anspruch 1, bei dem eine Ansteuerfrequenz der Ansteuerschaltung (15) in einem Bereich von 0,01 Hz bis 1 Hz liegt.

**10.** Selbstverdunkelnder Schweißhelm mit dem selbstverdunkelnden Filter nach einem der Ansprüche 1 bis 9.

**Revendications**

**1.** Filtre auto-obscurcissant (1), comprenant :

une première feuille de cristaux liquides (11) ;
deux feuilles polarisantes (13) qui comprennent une première feuille polarisante (131) et une deuxième feuille polarisante (133) et qui sont agencées séparément sur deux côtés de la première feuille de cristaux liquides (11) ;
un circuit de commande (15) commandant la première feuille de cristaux liquides (11) ; **caractérisé par**
un dispositif de réglage d'angle (17) ;
au moins l'une des deux feuilles polarisantes (13) étant réalisée sous forme de feuille polarisante mobile rotative (133) pour modifier un angle entre les axes de transmission des deux feuilles polarisantes (13), l'angle étant réglé par le dispositif de réglage d'angle (17) ;
la forme de la feuille polarisante mobile rotative (13) étant adaptée à la forme de la première feuille de cristaux liquides (11) de sorte que la feuille polarisante mobile rotative (13) ferme entièrement la première feuille de cristaux liquides (11) lorsque la feuille polarisante mobile rotative (13) est la deuxième feuille polarisante (133), et la deuxième feuille polarisante (133) tourne.

**2.** Filtre auto-obscurcissant selon la revendication 1, **caractérisé en ce que**, une position initiale de la feuille polarisante mobile rotative étant une position dans laquelle les sens des axes de transmission des deux feuilles polarisantes (13) sont perpendiculaires ou parallèles l'un à l'autre, et avec la position initiale en tant que référence, un angle de rotation de la feuille polarisante mobile rotative est de - 5° ~5°.

**3.** Filtre auto-obscurcissant selon la revendication 2, **caractérisé en ce que**, une position initiale de la feuille polarisante mobile rotative étant une position dans laquelle les sens des axes de transmission des deux feuilles polarisantes (13) sont perpendiculaires ou parallèles l'un à l'autre, et avec la position initiale en tant que référence, un angle de rotation de la feuille polarisante mobile rotative est de 0° - 5°.

**4.** Filtre auto-obscurcissant selon la revendication 1, comprenant en outre :

une deuxième feuille de cristaux liquides (11), les première et deuxième feuilles de cristaux liquides (11) étant agencées en parallèle, et
une troisième feuille polarisante (13),
la première feuille de cristaux liquides (11) étant prise en sandwich entre la première feuille polarisante (131) et la deuxième feuille polarisante (132), et la deuxième feuille de cristaux liquides (11) étant prise en sandwich entre la deuxième feuille polarisante (133) et la troisième feuille polarisante (135),

la première feuille polarisante (131) et/ou la deuxième feuille polarisante (132) et/ou la troisième feuille polarisante (135) étant la feuille polarisante mobile rotative.

5. Filtre auto-obscurcissant selon la revendication 2, **caractérisé en ce que** le dispositif de réglage d'angle (17) comprend :

un bouton de réglage d'angle (171) ;
une came (173) en forme de disque reliée au bouton de réglage d'angle (171) ; et
un élément élastique (175) ;
la came (173) en forme de disque butant contre un côté de la feuille polarisante mobile rotative, et l'élément élastique (175) étant relié à l'autre côté de la feuille polarisante mobile rotative.

6. Filtre auto-obscurcissant selon la revendication 5, **caractérisé en ce que** l'élément élastique (175) est un ressort.

7. Filtre auto-obscurcissant selon la revendication 1, un facteur de transmission de la lumière visible du filtre auto-obscurcissant (1) étant dans une plage de $10^{-6}$ à $10^{-1}$.

8. Filtre auto-obscurcissant selon la revendication 1, une tension de commande du circuit de commande (15) étant dans une plage de 20V à 40V.

9. Filtre auto-obscurcissant selon la revendication 1, une fréquence de commande du circuit de commande (15) étant dans une plage de 0,01 Hz à 1 Hz.

10. Casque de soudage auto-obscurcissant, comprenant le filtre auto-obscurcissant selon l'une des revendications 1 à 9.

$\underset{\sim}{1}$

133    131    11

175    173    171

17

FIG. 1

$\underset{\sim}{1}$

FIG. 2

EP 3 617 779 B1

FIG. 3

15

FIG. 4

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5515186 A
- US 5405184 A
- US 2004011603 A1